(19) Europäisches Patentamt / European Patent Office / Office européen des brevets

(11) **EP 4 736 816 A2**

(12) # DEMANDE DE BREVET EUROPEEN

(43) Date de publication:
**06.05.2026 Bulletin 2026/19**

(21) Numéro de dépôt: **26166741.4**

(22) Date de dépôt: **27.02.2024**

(51) Classification Internationale des Brevets (IPC):
**A61F 2/16** *(2006.01)*

(52) Classification Coopérative des Brevets (CPC):
**G16H 20/40; A61F 2/16; G16H 50/20; G16H 50/30;
G16H 50/70**

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priorité: **27.02.2023 FR 2301764**

(62) Numéro(s) de document de la (des) demande(s)
initiale(s) en application de l'article 76 CBE:
**24159882.0 / 4 421 818**

(71) Demandeur: **PromTime SAS**
**75008 Paris (FR)**

(72) Inventeur: **KATZ, Gregory**
**75116 Paris (FR)**

(74) Mandataire: **Ipsilon**
**12 Avenue d'Italie**
**75013 Paris (FR)**

Remarques:
Cette demande a été déposée le 23.03.2026 comme
demande divisionnaire de la demande mentionnée
sous le code INID 62.

(54) **PROCÉDÉ DE COMPARAISON D' IMPLANTS OU DE PROTHÈSES**

(57) Procédé de comparaison d'implants ou de prothèses, et/ou de contextes opératoires, à partir d'une population d'individus portant chacun un implant ou une prothèse, le procédé comportant :

A) pour chaque individu:
- renseignement du type d'implants ou de prothèses, et/ou d'au moins un paramètre du contexte opératoire de mise en place de l'implant ou de la prothèse;
- acquisition de paramètres cliniques; et
- détermination d'un score (GS) quantifiant un bénéfice apporté à l'individu par l'intervention;

B) classification des individus de sorte à obtenir au moins deux catégories de profil de risque indicatives d'un rapport entre un bénéfice apporté et le risque encouru;
C) pour chaque catégorie, calcul d'un GS de référence, le GS de référence étant un quantile des GS des individus de la catégorie ;
après l'étape C), comparaison, au sein de chaque catégorie, des types d'implants ou de prothèses et/ou des contextes opératoires en fonction des GS.

[Fig 1]

| A) Acquisition données |
|---|
| B) Classification des individus en catégories de profil de risque |
| C) Calcul des GS de référence |

Fig. 1

EP 4 736 816 A2

**Description**

**Domaine technique**

La présente invention concerne des procédés de comparaison du bénéfice apporté par un dispositif médical tel qu'un implant, par exemple un implant intraoculaire en ophtalmologie, une prothèse de hanche en orthopédie, une audioprothèse en audiologie, une prothèse dentaire en odontologie, ou un stent en cardiologie, à un individu et plus généralement de comparaison de contextes opératoires. L'invention concerne également des dispositifs pour mettre en œuvre ces procédés.

**Technique antérieure**

L'évaluation d'une intervention médicale nécessite de prendre en compte de nombreux facteurs. Il n'est pas aisé de comparer en vie réelle le bénéfice clinique des dispositifs médicaux selon les différents parcours de soins et la diversité des profils de patients. En effet, chaque intervention médicale dépend du praticien qui la réalise, de son expérience, du cadre dans lequel elle est effectuée, des outils à sa disposition mais également du patient, de son état physique, de ses antécédents, de son hygiène de vie, de ses comorbidités.

De plus, de façon générale, la réalisation d'une intervention médicale entraine systématiquement des risques, même minimes, pour le patient. Cependant, la réalisation d'une intervention médicale n'entraîne pas toujours les résultats escomptés pour le patient, quand bien même l'intervention médicale serait, d'un point de vue médical, considérée comme réussie. En particulier, chaque patient étant différent, les résultats d'une intervention médicale ou chirurgicale réalisée par un praticien diffèrent d'un patient à l'autre selon les pratiques et produits de santé utilisés, mais surtout selon les profils des patients eux-mêmes.

Enfin, n'est actuellement pas rare de réaliser certaines interventions médicales, de façon quasi-systématique, en général par précaution ou par simplicité pour le praticien, sans réelle nécessité. En effet, l'Organisation Mondiale de la Santé et l'Organisation de Coopération et de Développement Economiques indiquent qu'environ 30% des actes médicaux ne sont pas pertinents, c'est-à-dire qu'ils n'apportent aucun bénéfice clinique pour le patient, autrement dit qu'il s'agit de soins gaspillés susceptibles de générer des complications médicales évitables pour le patient, et des dépenses de santé évitables pour les systèmes de soins.

Par ailleurs, la comparaison des résultats en vie réelle diffère de façon notable des résultats d'essais cliniques randomisés contrôlés, publiés dans la littérature scientifique, car la vie réelle comporte des contingences que les protocoles d'essais cliniques contrôlent, avec l'interaction de nombreux facteurs exogènes tels que l'observance du patient, le respect des bonnes pratiques par le praticien, etc. Il est classique d'observer que les résultats d'études cliniques ne sont pas nécessairement transposés dans les résultats de la vie réelle du fait de son hétérogénéité, par exemple en termes de conditions d'utilisation des produits de santé, mais aussi des comportements des patients et des soignants.

En conséquence, il existe un besoin permettant de comparer les interventions médicales entre elles, notamment pour comparer la pose d'implants ou prothèses, par exemple d'implants intraoculaires pour la cataracte, des prothèses orthopédiques pour l'arthrose de la hanche ou du genou, ou des audioprothèses pour les malentendants ou encore des stents pour les patients atteints de coronaropathies, et en particulier pour comparer des types d'implants ou de prothèses. Il existe également un intérêt à limiter des interventions médicales s'avérant inutiles pour les patients. Enfin, il existe un besoin permanent pour aider les praticiens à planifier les interventions médicales.

**Exposé de l'invention**

L'invention vise à répondre à tout ou partie de ces besoins et elle y parvient, selon l'un de ses aspects, grâce à un procédé de comparaison d'implants ou de prothèses, et/ou de contextes opératoires de mise en place de l'implant ou de la prothèse, à partir d'une population d'individus portant chacun un implant, par exemple un implant intraoculaire, une prothèse orthopédique, une audioprothèse, ou un stent, le procédé de comparaison comportant les étapes suivantes :

A) pour chaque individu de la population d'individus :

- renseignement du type d'implants ou de prothèses, et/ou d'au moins un paramètre du contexte opératoire de mise en place de l'implant ou de la prothèse;
- acquisition de paramètres cliniques; et
- détermination d'un score de « gain de santé » (GS) quantifiant un bénéfice apporté à l'individu par l'intervention médicale;

B) classification des individus de la population d'individus de sorte à obtenir au moins deux catégories de profil de risque indicatives d'un rapport entre un bénéfice apporté par une intervention médicale et le risque encouru par la mise en œuvre d'une intervention médicale, la classification regroupant les individus en fonction de leur gain de santé et de leurs paramètres cliniques, notamment en fonction des paramètres cliniques ayant un impact négatif sur le gain de santé, la classification étant en particulier effectuée au moyen d'une régression statistique, notamment une régression linéaire multivariée ;

C) pour chaque catégorie de profil de risque, calcul d'un gain de santé de référence, le gain de santé de référence étant un quantile, notamment la moyenne ou la médiane, ou une mesure proportionnelle à la différence minimale d'importance clinique (« MCID ») des gains de santé des individus de la catégorie, ou toute combinaison de ceux-ci ;

le procédé comportant, après l'étape C), la comparaison, de préférence au sein de chaque catégorie de profils de risque, des types d'implants ou de prothèses et/ou des contextes opératoires en fonction des gains de santé.

Par intervention médicale, il faut comprendre l'administration chirurgicale de l'implant lorsqu'on considère un implant ou la pose d'une prothèse lorsqu'on considère une prothèse.

Le gain de santé peut permettre de quantifier l'amélioration de la qualité de vie de patient générée par l'intervention médicale.

Alternativement ou additionnellement, la comparaison peut être réalisées en considérant la population d'individus dans son ensemble.

Un procédé selon l'invention permet avantageusement de comparer rapidement, précisément et simplement des interventions médicales entre elles, c'est-à-dire de hiérarchiser les scores de gains de santé associés à ces interventions médicales et en particulier aux types d'implants ou de prothèses au sein d'un groupe d'individus statistiquement homogènes. En particulier, pour une catégorie donnée, il est possible de comparer les implants par rapport au gain de santé de référence, et ainsi déterminer si l'intervention médicale pour un individu est avantageuse par rapport à des individus ayant le même profil. On peut facilement déterminer quel type d'implant ou de prothèse est plus efficace pour une catégorie donnée.

Il est également possible de déterminer en fonction d'un gain de santé calculé si un implant est pertinent pour des individus d'une catégorie. Par exemple si pour un type d'implants donné tous les patients de la catégorie portant ce type d'implants ont un gain de santé inférieur au gain de santé de référence de la catégorie, alors le type d'implant n'est pas adapté pour les individus de ladite catégorie.

La comparaison après l'étape C) peut comprendre un ordonnancement des types d'implants, respectivement de prothèses, et/ou des contextes opératoires, en particulier des types d'implants, respectivement de prothèses, et des contextes opératoires, en fonction du gain de santé, pour chacune des catégories considérées séparément.

La comparaison peut être présentée en étant affichée sur un écran ou imprimée sur un support.

Un procédé de comparaison selon l'invention peut comporter les étapes suivantes, après l'étape C) :

D) pour chaque catégorie de profil de risque, détermination d'un coefficient de pondération puis calcul d'un gain de santé de référence ajusté égal au produit du coefficient de pondération par le gain de santé de référence,
les coefficients de pondération étant choisis de sorte à obtenir un ensemble de gains de santé de référence ajustés sensiblement égaux entre eux;

E) pour chaque individu de la population d'individus, ajustement du gain de santé, déterminé à l'étape A), en multipliant le gain de santé par le coefficient de pondération déterminé pour la catégorie à laquelle appartient l'individu, de sorte à obtenir un ensemble de gains de santé pondérés;

F) comparaison des types d'implants ou de prothèses, et/ou des contextes opératoires en comparant les gains de santé ajustés.

Ainsi, dans un mode de réalisation particulier de l'invention, le procédé de comparaison d'implants ou de prothèses, et/ou de contextes opératoires de mise en place de l'implant ou de la prothèse comporte les étapes suivantes :

A) pour chaque individu de la population d'individus :

-    renseignement du type d'implants ou de prothèses, et/ou d'au moins un paramètre du contexte opératoire de mise en place de l'implant ou de la prothèse;
-    acquisition de paramètres cliniques; et
-    détermination d'un score de gain de santé quantifiant un bénéfice apporté à l'individu par l'intervention médicale ;

B) classification des individus de la population d'individus de sorte à obtenir au moins deux catégories de profil de risque indicatives d'un rapport entre un bénéfice apporté par une intervention médicale et le risque encouru par la mise en œuvre d'une intervention médicale, la classification regroupant les individus en fonction de leur gain de santé et de leurs paramètres cliniques, notamment en fonction des paramètres cliniques ayant un impact négatif sur le gain de santé, la classification étant en particulier effectuée au moyen d'une régression statistique, notamment une régression linéaire multivariée ;

C) pour chaque catégorie de profil de risque, calcul d'un gain de santé de référence, le gain de santé de référence étant un quantile, notamment la moyenne ou la médiane, ou une mesure proportionnelle à la différence minimale d'importance clinique (« MCID ») des gains de santé des individus de la catégorie, ou toute combinaison de celles-ci ;

D) pour chaque catégorie de profil de risque, détermination d'un coefficient de pondération puis calcul d'un gain de santé de référence ajusté égal au produit du coefficient de pondération par le gain de santé de référence, les coefficients de pondération étant choisis de sorte à obtenir un ensemble de gains de santé de référence ajustés sensiblement égaux entre eux;

E) pour chaque individu de la population d'individus, ajustement du gain de santé, déterminé à l'étape A), en multipliant le gain de santé par le coefficient de pondération déterminé pour la catégorie à laquelle appartient l'individu, de sorte à obtenir un ensemble de gains de santé pondérés;

F) comparaison des types d'implants ou de prothèses, et/ou des contextes opératoires en comparant les gains de santé ajustés.

En particulier, le contexte opératoire peut comporter le nom d'un praticien, la durée d'expérience du praticien, sa formation, la technique opératoire, l'hôpital ou la clinique où l'intervention médicale a lieu ou le département ou la commune où l'intervention médicale a lieu, en particulier la précision de si l'intervention médicale a lieu dans un désert médical ou non, un ou des dispositifs médicaux utilisés lors de l'intervention (autres que l'implant lui-même ou la prothèse elle-même).

Le procédé de comparaison d'implants ou de prothèses permet en particulier de comparer les bénéfices apportés par différents types d'implants ou de prothèses respectivement, indépendamment de la catégorie considérée.

Le procédé de comparaison selon l'invention permet avantageusement, en fonction des profils de patients, de déterminer les types d'implants, respectivement de prothèses, qui optimisent la qualité de vie des patients dans leurs activités quotidiennes.

Le procédé de comparaison permet, selon un autre de ses aspects, d'identifier les contextes opératoires les plus pertinents.

Deux implants de même type sont des implants identiques ou éventuellement qui diffèrent par leur taille, étant adaptés par exemple à des personnes ayant des yeux plus ou moins grands.

Deux prothèses de même type sont des prothèses identiques ou éventuellement qui diffèrent par leur taille, étant adaptées par exemple à des personnes de plus ou moins grandes tailles.

Le type d'implant, respectivement de prothèses, peut être caractérisé par sa forme, ses propriétés, les matériaux utilisés, son mode d'action ou d'administration.

De préférence, les implants sont des implants pour traiter la cataracte.

Les prothèses peuvent être des audioprothèses, des prothèses orthopédiques, des prothèses mammaires, des prothèses dentaires. De préférence, les prothèses sont des prothèses de hanche et de genou.

A l'étape A), pour chaque individu de la population d'individus, le gain de santé peut être déterminé par comparaison d'un indicateur représentatif d'un état pré-interventionnel de l'individu avec un indicateur représentatif d'un état post-interventionnel de l'individu.

Les indicateurs pré-interventionnels et/ou post-interventionnels, de préférence, les indicateurs pré-interventionnels et post-interventionnels, peuvent être des mesures du résultat de l'intervention rapporté par le patient exprimés en termes de qualité de vie ou de score fonctionnel, dits indicateurs « PROM » (« Patient-reported outcomes measures » en anglais) ou des mesures de l'expérience de l'intervention rapportée par le patient exprimée par exemple en termes de qualité relationnelle avec le soignant (ou PREM, « Patient-reported experience measures » en anglais).

De préférence, le ou les indicateurs pré-interventionnels et post-interventionnels sont déterminés au moyen d'au moins un questionnaire, en particulier renseigné par l'individu.

Le questionnaire peut être un questionnaire générique. Il peut comporter des questions portant sur : la qualité de vie de l'individu, l'impact d'une pathologie sur la vie de l'individu, une partie du corps, la douleur, un état de santé, le contexte familial, la satisfaction de soins reçus, la satisfaction d'informations reçues, et/ou l'expérience opératoire vécue.

Le questionnaire peut avantageusement être renseigné à distance par chaque individu, par exemple via une interface digitale, puis être stocké dans une base de données.

A partir du questionnaire comportant plusieurs questions, un unique indicateur pré-interventionnel, ou post-interventionnel, peut être calculé.

Les réponses aux questions peuvent être analysées au moyen d'une analyse psychométrique, notamment de type analyse de Rasch. Avantageusement les réponses aux questions sont ainsi normalisées.

Dans un mode de réalisation particulier, les réponses aux questions peuvent être validées, par exemple par un assistant médical ou un praticien.

L'indicateur pré-interventionnel et l'indicateur post-interventionnel peuvent être un pourcentage, un chiffre ou un nombre par exemple compris entre 0 et 100.

Le gain de santé peut être la différence entre l'indicateur post-interventionnel et l'indicateur pré-interventionnel. Le gain de santé peut être un pourcentage, un chiffre ou un nombre, par exemple compris entre -100 et 100.

Le gain de santé de chaque individu peut être stocké dans une base de données, de préférence après anonymisation.

L'acquisition des paramètres cliniques peut être effectuée par un assistant médical, un praticien, par l'individu ou par recherche dans un dossier médical, par exemple stocké dans une base de données générale d'un hôpital ou d'une clinique.

Les paramètres cliniques peuvent être choisis parmi : l'âge, le sexe, la présence de maladies chroniques, de diabète, d'allergies, de comorbidités, en particulier de comorbidités oculaires, un degré de sévérité de ces comorbidités, le nombre d'interventions médicales antérieures, la nature d'interventions médicales antérieures, par exemple interventions ophtalmiques antérieures. Les paramètres cliniques peuvent en outre comporter des paramètres socio-démographiques.

A l'étape B), la classification peut être mise en œuvre par tout algorithme de classification connu. L'algorithme prend en entrée au moins les gains de santé et les paramètres cliniques, et fournit en sortie au moins deux catégories de profil de risque, mieux encore, au moins trois catégories de profil de risque.

Dans un cas particulier, l'algorithme fournit en sortie exactement trois catégories de profil de risque : une première catégorie comportant les individus ayant un profil de risque sévère, une deuxième catégorie comportant les individus ayant un profil de risque intermédiaire et une troisième catégorie comportant les individus ayant un profil de risque faible.

L'objectif de cette classification est de déterminer des catégories distinguant les individus en fonction d'un rapport entre le bénéfice apporté à l'individu par une intervention médicale et le risque clinique qu'il encourt. En d'autres termes, le but de cette classification est d'identifier les profils pour lesquels le bénéfice apporté par l'intervention médicale est supérieur au risque encouru par l'intervention.

L'algorithme peut être un algorithme de centres mobiles (ou « k-means »), arbre de décision, analyse en composante principale, méthode des plus proches voisins.

De préférence, elle est effectuée au moyen d'une régression statistique.

La classification peut être effectuée au moyen d'une régression linéaire multivariée, les variables explicatives de la régression étant tout ou partie des paramètres cliniques.

Parallèlement à la classification, à l'étape B) un algorithme de sélection de caractéristiques peut être mis en œuvre, identifiant les paramètres cliniques ayant un impact négatif sur le gain de santé et/ou les paramètres cliniques ayant un impact positif sur le gain de santé.

A l'étape D), les coefficients de pondération sont choisis de sorte que :

$$[\text{Math 1}]$$
$$\forall i \in [\![1,N]\!], \forall k \in [\![1,N]\!], k \neq i,$$

$$c_i * GS_{r\acute{e}f,i} = c_k * GS_{r\acute{e}f,k} + \varepsilon \Leftrightarrow GS_{ajust\acute{e},i} = GS_{ajust\acute{e},k} + \varepsilon$$

$GS_{r\acute{e}f,i}$, étant le gain de santé de référence de la catégorie i, $GS_{ajust\acute{e},i}$ étant le gain de santé ajusté de la catégorie i, N étant le nombre de catégories déterminées à l'étape B), $c_i$ étant le coefficient de pondération de la catégorie i, $\varepsilon$ étant inférieur à 10% du maximum des gains de santé de référence, mieux inférieur à 5% du maximum des gains de santé de référence, mieux inférieur à 2% du maximum des gains de santé de référence, mieux inférieur à 1% du maximum des gains de santé de référence encore mieux $\varepsilon$ est nul.

A l'étape F), la comparaison peut être réalisée en comparant les gains de santé ajustés en fonction des paramètres cliniques ayant un impact négatif sur le gain de santé.

Alternativement ou additionnellement, la comparaison peut être réalisée en comparant les gains de santé ajustés en fonction des paramètres cliniques ayant un impact positif sur le gain de santé.

La comparaison peut comprendre un ordonnancement des types d'implants, respectivement de prothèses, et/ou des contextes opératoires, en particulier des types d'implants, respectivement de prothèses, et des contextes opératoires, en fonction du gain de santé ajusté.

La comparaison peut être présentée en étant affichée sur un écran ou imprimée sur un support.

L'invention permet de comparer les interventions médicales effectuées indépendamment des individus et des caractéristiques propres à chaque individu et à chaque intervention, en particulier à chaque praticien.

La détermination du gain de santé ajusté permet avantageusement de comparer rapidement, simplement et précisément les types d'implants ou de prothèses et/ou des contextes opératoires à l'aide d'un unique critère.

L'invention permet de revaloriser les interventions complexes mais nécessaires, par rapport à des interventions de routine non nécessaires qui présenteraient initialement un gain de santé similaire au gain de santé résultant de l'intervention médicale complexe, et ainsi éviter la réalisation d'intervention non pertinente.

L'invention porte également sur un procédé d'aide à la sélection d'un type d'implants ou de prothèses, et/ou d'un contexte opératoire, pour planifier une intervention médicale pour un patient, le procédé d'aide à la sélection comportant les étapes suivantes :

1) renseignement de résultats d'une comparaison de types d'implants ou de prothèses pour une catégorie d'individu donnée, et/ou de contextes opératoires de mise en place de ces implants, respectivement prothèses, de préférence les résultats étant issus d'un procédé de comparaison selon invention ;
2) seuillage des résultats issus de l'étape 1) de sorte à sélectionner le ou les types d'implants, respectivement prothèses, et/ou le ou les contextes opératoires pour lesquels les gains de santé sont supérieurs à une valeur seuil prédéterminée.

A l'étape 2), plusieurs types d'implants, respectivement prothèses, peuvent être sélectionnés. Il est également possible qu'aucun type d'implants, respectivement prothèses, ne soit sélectionné. Lorsqu'aucun type d'implants, respectivement prothèses, n'est sélectionné, la pertinence de l'intervention médicale peut être remise en question.

De même, lorsque le procédé porte sur une aide à la sélection de contextes opératoires, à l'étape 2), plusieurs contextes opératoires peuvent être sélectionnés ou aucun contexte opératoire peut être sélectionné.

Le procédé d'aide à la sélection peut comporter avant l'étape 2), le renseignement d'au moins un paramètre clinique du patient, l'étape 1) résultant de la mise en œuvre d'un procédé de comparaison selon l'invention, l'étape 2) comportant les étapes suivantes :

2.1) sélection de la catégorie de profil de risque en fonction du au moins un paramètre clinique du patient; puis,
2.2) seuillage des gains de santé de la catégorie sélectionnée à l'étape 2.1), en sélectionnant parmi les types d'implants ou les types de prothèses, et/ou les contextes opératoires, les types d'implants et/ou les contextes opératoires, respectivement les types de prothèses et/ou les contextes opératoires, associés à un gain de santé supérieur à une valeur seuil prédéterminée.

Le procédé d'aide à la sélection peut en particulier être utilisé pour sélectionner un type d'implants intraoculaires pour une implantation en particulier de traitement de la cataracte pour un patient. Dans ce cas particulier, à l'étape 1), un procédé de comparaison du bénéfice apporté à un individu par un type d'implants intraoculaires est mis en œuvre, et à l'étape 2) les résultats sont seuillés de sorte à sélectionner les types d'implants intraoculaires pour lesquels les gains de santé sont supérieurs à la valeur seuil prédéterminée, l'étape 2) pouvant comporter 2.1) la sélection d'une catégorie de profil de risque pour laquelle les paramètres cliniques des individus correspondent à un paramètre clinique renseigné pour le patient, puis 2.2) le seuillage des gains de santé en sélectionnant parmi les types d'implants intraoculaires de la catégorie sélectionnée à l'étape 2.1), les types d'implants associés à un gain de santé supérieur à la valeur seuil prédéterminée.

Un procédé d'aide à la sélection peut également être utilisé pour sélectionner un type de prothèses, par exemple pour une prothèse de la hanche.

L'invention porte en outre sur un procédé de fabrication d'un implant ou d'une prothèse, comportant la mise en œuvre d'un procédé d'aide à la sélection d'un type d'implants ou de prothèses de sorte à sélectionner au moins un type d'implants ou de prothèses, puis la fabrication d'un implant ou de prothèse de même type que celui qui a été sélectionné, notamment identique.

Lorsque plusieurs types d'implants, respectivement de prothèses, sont sélectionnés lors de la mise en œuvre du procédé d'aide à la sélection, le type d'implants, respectivement de prothèse, fabriqué peut être celui correspondant au type d'implants, respectivement de prothèses, sélectionné ayant le gain de santé le plus élevé.

Alternativement, lorsque plusieurs types d'implants, respectivement de prothèses, sont sélectionnés lors de la mise en œuvre du procédé d'aide à la sélection, le praticien choisit le type d'implants, respectivement de prothèses, à faire fabriquer en vue de l'intervention.

De préférence, les résultats de l'étape 1) d'un procédé d'aide à la sélection selon l'invention comporte la mise en œuvre d'un procédé de comparaison selon l'invention comportant les étapes A) à F), l'étape 2) comportant :
2) seuillage des résultats issus de l'étape 1) de sorte à sélectionner le ou les types d'implants, respectivement prothèses, et/ou le ou les contextes opératoires pour lesquels les gains de santé ajustés sont supérieurs à une valeur seuil prédéterminée.

A l'étape 2), plusieurs types d'implant , respectivement prothèses, peuvent être sélectionnés. Il est également possible qu'aucun type d'implants, respectivement prothèses, ne soit sélectionné. Lorsqu'aucun type d'implants, respectivement prothèses, n'est sélectionné, la pertinence de l'intervention médicale peut être remise en question.

De même, lorsque le procédé porte sur une aide à la sélection de contextes opératoires, à l'étape 2), plusieurs contextes opératoires peuvent être sélectionnés ou aucun contexte opératoire peut être sélectionné.

La valeur seuil prédéterminée peut être déterminée par un praticien. Elle peut être choisie en accord avec le patient. Une valeur minimale de seuil peut être imposée par l'hôpital ou la clinique du praticien.

A l'étape 2), la valeur seuil peut être définie de sorte que le seuillage sélectionnera les gains de santés pour lesquels l'indicateur post-interventionnel est au moins 10% plus grand que l'indicateur pré-interventionnel, mieux au moins 20% plus important que l'indicateur pré-interventionnel, encore mieux au moins 30% plus important que l'indicateur pré-interventionnel.

Dans un mode de réalisation particulier, la valeur seuil est calculée en fonction de paramètres du patient pour lequel une intervention est planifiée.

Le procédé d'aide à la sélection peut comporter avant l'étape 2), le renseignement d'au moins un paramètre clinique du patient, l'étape 1) résultant de la mise en œuvre d'un procédé de comparaison selon l'invention, l'étape 2) comportant les étapes suivantes :

2.1) présélection des types d'implants ou de prothèses, et/ou de contextes opératoires, pour lesquels les paramètres cliniques des individus de la population d'individus correspondent au paramètre clinique renseigné pour le patient; puis,

2.2) seuillage des gains de santé ajustés, en sélectionnant parmi les types d'implants ou les types de prothèses, et/ou les contextes opératoires, présélectionnés à l'étape 2.1), les types d'implants et/ou les contextes opératoires, respectivement les types de prothèses et/ou les contextes opératoires, associés à un gain de santé ajusté supérieur à une valeur seuil prédéterminée.

Le procédé d'aide à la sélection peut en particulier être utilisé pour sélectionner un type d'implants intraoculaires pour une implantation en particulier de traitement de la cataracte pour un patient. Dans ce cas particulier, à l'étape 1), un procédé de comparaison du bénéfice apporté à un individu par un type d'implants intraoculaires est mis en œuvre, et à l'étape 2) les résultats sont seuillés de sorte à sélectionner les types d'implants intraoculaires pour lesquels les gains de santé ajustés sont supérieurs à la valeur seuil prédéterminée, l'étape 2) pouvant comporter 2.1) la présélection d'implants intraoculaires pour lesquels les paramètres cliniques des individus correspondent à un paramètre clinique renseigné pour le patient, puis 2.2) le seuillage des gains de santé ajustés en sélectionnant parmi les types d'implants intraoculaires présélectionnés à l'étape 2.1), les types d'implants associés à un gain de santé ajusté supérieur à la valeur seuil prédéterminée.

Un procédé d'aide à la sélection peut également être utilisé pour sélectionner un type de prothèses, par exemple pour une prothèse de la hanche.

L'invention porte en outre sur un procédé de fabrication d'un implant ou d'une prothèse, comportant la mise en œuvre d'un procédé d'aide à la sélection d'un type d'implants ou de prothèses de sorte à sélectionner au moins un type d'implants ou de prothèses, puis la fabrication d'un implant ou de prothèse de même type que celui qui a été sélectionné, notamment identique.

Lorsque plusieurs types d'implants, respectivement de prothèses, sont sélectionnés lors de la mise en œuvre du procédé d'aide à la sélection, le type d'implants, respectivement de prothèse, fabriqué peut être celui correspondant au type d'implants, respectivement de prothèses, sélectionné ayant le gain de santé ajusté le plus élevé.

Alternativement, lorsque plusieurs types d'implants, respectivement de prothèses, sont sélectionnés lors de la mise en œuvre du procédé d'aide à la sélection, le praticien choisit le type d'implants, respectivement de prothèses, à faire fabriquer en vue de l'intervention.

L'invention porte également sur un système de traitement de données comportant :

une base de données stockant pour une population d'individus portant chacun un implant ou une prothèse : au moins un type d'implants ou de prothèse, et/ou un paramètre de contexte opératoire, en particulier choisi parmi le nom d'un praticien, la durée d'expérience du praticien, sa formation, la technique opératoire, l'hôpital ou la clinique où l'intervention a lieu, un ou des dispositifs médicaux utilisés lors de l'intervention (autres que l'implant lui-même ou la prothèses elle-même);

un programme d'ordinateur comprenant des instructions qui, lorsque le programme est exécuté par un ordinateur, conduisent celui-ci à mettre en œuvre les étapes B) et C), de préférence les étapes B) à F), d'un procédé de comparaison selon l'invention, et/ou les étapes 1) et 2) d'un procédé d'aide à la sélection, en particulier d'un type d'implants ou d'un type de prothèses.

Le système de traitement de données peut comporter des moyens de communication permettant de recueillir les types d'implants ou les types de prothèses, et/ou les contextes opératoires, les paramètres cliniques et les gains de santé pour la mise en œuvre de l'étape A) d'un procédé de comparaison selon l'invention.

Les moyens de communication peuvent être des moyens de communication digitale enrichissant la base de données à partir de renseignement fournis via une interface digitale.

## Brève description des dessins

L'invention pourra être mieux comprise à la lecture de la description détaillée qui va suivre, d'exemples de mise en œuvre non limitatifs de celle-ci, et à l'examen du dessin annexé, sur lequel :

[Fig 1] représente les étapes d'un procédé de comparaison d'implants et/ou de contextes opératoires selon l'invention ;
[Fig 2] représente les étapes d'un procédé de comparaison d'implants et/ou de contextes opératoires selon l'invention ;
[Fig 3] représente les étapes d'un procédé de fabrication d'un implant selon l'invention ;
[Fig 4] est un schéma bloc d'un système de traitement de données selon l'invention ;
[Fig 5] est un schéma bloc d'un dispositif pour mettre en œuvre un procédé de fabrication d'un implant;
[Fig 6] est un tableau illustrant une classification issue de la mise en œuvre d'une étape B);
[Fig 7] représente des boîtes à moustaches des catégories issues de la classification illustrée à la figure 6 ; et
[Fig 8] est un tableau illustrant une sélection de paramètres cliniques ayant un impact négatif sur le gain de santé.

## Description détaillée

Les figures portent sur des procédés de comparaison et de fabrication, et des systèmes pour la mise en œuvre de ces procédés pour des implants, en particulier des implants intraoculaires.

L'invention n'est pas limitée à ce cas particulier. En particulier, un développement similaire peut être réalisé pour des prothèses et en particulier des prothèses de la hanche.

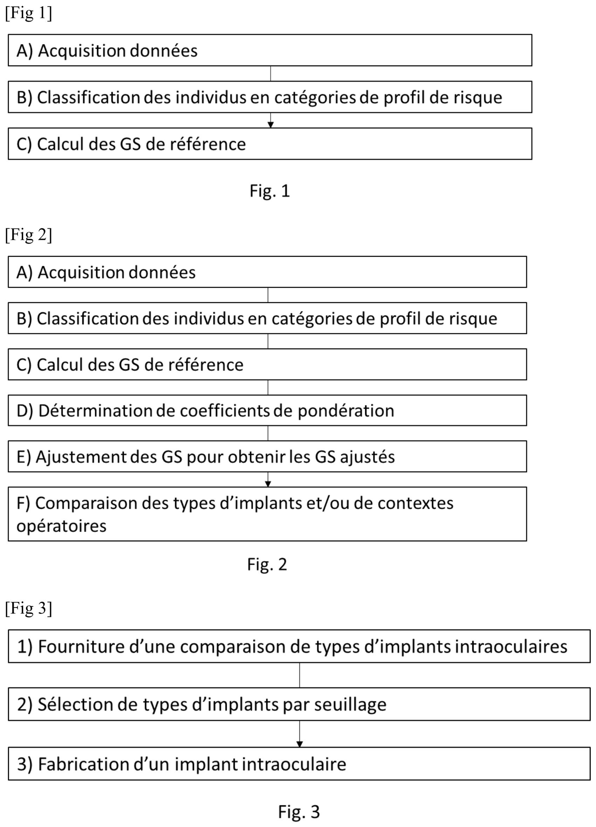

La figure 1 illustre les différentes étapes d'un procédé de comparaison d'implants et/ou de contextes opératoires selon l'invention.

Acquisition de données

L'étape A) porte sur une acquisition de données relatives à une population d'individus, comportant N individus.

Pour chaque individu k, k appartenant à N, les paramètres comportent au moins un type d'implants et/ou un paramètre du contexte opératoire de l'intervention médicale, autrement dit l'implantation, des paramètres cliniques ($\{C_{k,1} ; C_{k;2} ; .. ; C_{k,M}\}$) et un gain de santé ($GS_k$), l'intervention ayant eu lieu précédemment.

Le paramètre du contexte opératoire de mise en place de l'implant peut comporter le nom d'un praticien, la durée d'expérience du praticien, sa formation, la technique opératoire, l'hôpital ou la clinique où l'intervention médicale a lieu ou le département ou la commune où l'intervention médicale a lieu, en particulier la précision de si l'intervention médicale a lieu dans un désert médical ou non, un ou des dispositifs médicaux utilisés lors de l'intervention (autres que l'implant lui-même).

Dans un mode de réalisation particulier, plusieurs paramètres du contexte opératoire sont renseignés.

Dans le cas particulier d'un procédé de comparaison du bénéfice de l'apport d'un implant intraoculaire à un individu, les paramètres comportent au moins un type d'implants intraoculaires ($I_k$), de préférence un type d'implants intraoculaires pour traiter la cataracte, des paramètres cliniques ($\{C_{k,1} ; C_{k;2} ; .. ; C_{k,M}\}$) et un gain de santé ($GS_k$).

Les paramètres cliniques peuvent comporter : l'âge, le sexe, la présence de maladies oculaires diabétiques, la présence d'œdèmes maculaires diabétiques, la présence de glaucomes, la présence d'une dégénérescence maculaire, la présence d'autres comorbidités oculaires, un degré de sévérité de ces comorbidités, la réalisation d'opérations antérieures, en particulier d'opération d'un ou des deux yeux, la présence de pathologies héréditaires, la présence d'antécédents familiaux.

Le gain de santé pour un individu k ($GS_k$) est en particulier déterminé par comparaison d'un indicateur post-interventionnel ($ind_{post-int,k}$), représentatif d'un état post-interventionnel de l'individu k et d'un indicateur pré-interventionnel ($ind_{pré-int,k}$), représentatif d'un état pré-interventionnel de l'individu k. Par exemple, le gain de santé peut être calculé comme suit

$$GS_k = ind_{post-int,\, k} - ind_{pré-int,\, k}.$$

L'indicateur post-interventionnel et/ou l'indicateur pré-interventionnel, de préférence l'indicateur post-interventionnel et l'indicateur pré-interventionnel, peuvent être déterminés au moyen de questionnaires, en particulier au moyen de questionnaires PROM et/ou PREM, de préférence de questionnaires PROM et/ou PREM spécifiques à la cataracte.

Les questionnaires peuvent être renseignés par l'individu, sans intervention d'un praticien.

Les questionnaires peuvent être renseignés via une interface digitale, par exemple un questionnaire via Internet, un téléphone portable, une tablette, un ordinateur, les résultats des questionnaires étant alors transmis via des communications digitales à une base de données stockant les résultats.

Le gain de santé peut être un nombre ou un chiffre, par exemple compris entre - 100 et 100, un gain de santé négatif correspondant à un état d'un individu dégradé par l'intervention médicale.

Les paramètres peuvent être acquises indépendamment les unes des autres, en particulier elles peuvent être renseignées à des instants différents et par différents opérateurs.

Les paramètres peuvent être stockées dans une base de données 10.

Classification

A l'étape B), une classification est effectuée à partir des paramètres de l'étape A).

De préférence, une régression statistique est réalisée permettant de définir au moins deux catégories de profil de risque, de préférence au moins trois catégories de profil de risque.

Les catégories de profil de risque sont indicatives d'un rapport entre un bénéfice apporté par une intervention médicale et le risque encouru par la mise en œuvre d'une intervention médicale, la classification regroupant les individus en fonction de leur gain de santé et de leurs paramètres cliniques.

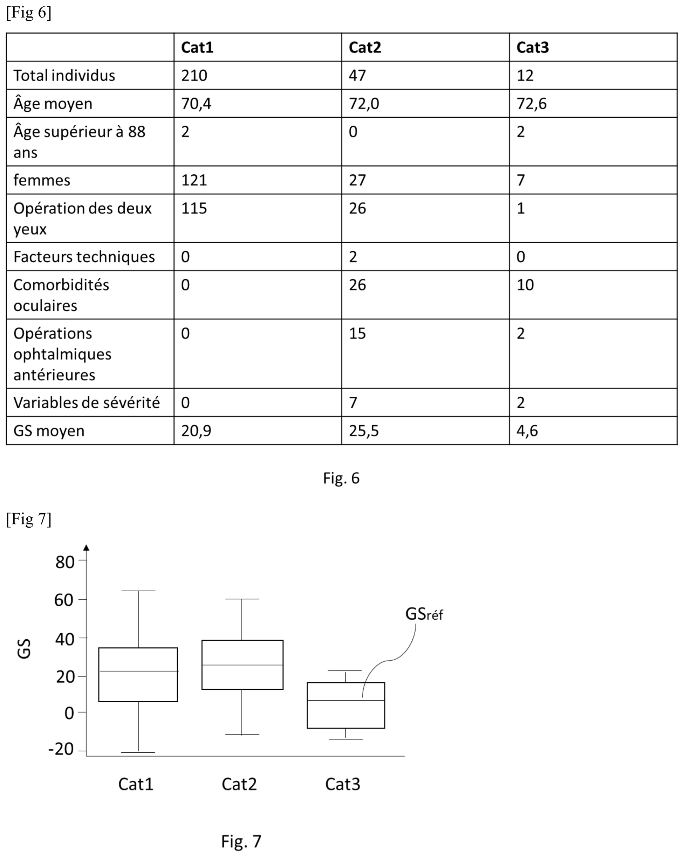

Un exemple de classification fournissant trois catégories de profil de risque est synthétisé dans le tableau de la figure 6 et le graphe de la figure 7.

Dans l'exemple des figures 6 et 7, les catégories de profil de risque distinguent les individus de la population en :

une première catégorie dont les paramètres cliniques n'indiquent a priori pas de difficulté de récupération ou pour l'intervention médicale;
une deuxième catégorie dont le gain de santé est relativement élevé et les paramètres cliniques comportent au moins un paramètre indiquant des potentielles difficultés de récupération et/ou pour l'intervention médicale;
une troisième catégorie dont le gain de santé est relativement faible et les paramètres cliniques comportent au moins un paramètre indiquant des difficultés potentielles de récupération et/ou pour l'intervention médicale.

Dans l'exemple des figures 6 et 7, N (nombre d'individus dans la population) est égal à 269, la première catégorie de profil de risque comporte 210 individus, la deuxième catégorie de profil de risque comporte 47 individus et la troisième catégorie de profil de risque comporte 12 individus.

La figure 7 représente la répartition des individus au sein de chacune des trois catégories en fonction de leur gain de santé. En particulier, la figure 7 permet de mettre en évidence le gain de santé moyen, le gain de santé minimum, le gain de santé maximum, le 1er quartile et le 3ème quartile des gains de santé, pour chaque catégorie.

La classification peut permettre d'identifier, au moyen d'un algorithme de sélection de caractéristiques, les paramètres cliniques ayant un impact négatif sur le gain de santé. En particulier, la principale différence entre la deuxième catégorie de profil de risque et la troisième catégorie de profil de risque de l'exemple illustré dans les figures 6 et 7 est la présence de paramètres cliniques impactant négativement le gain de santé chez les individus de la troisième catégorie de profil de risque.

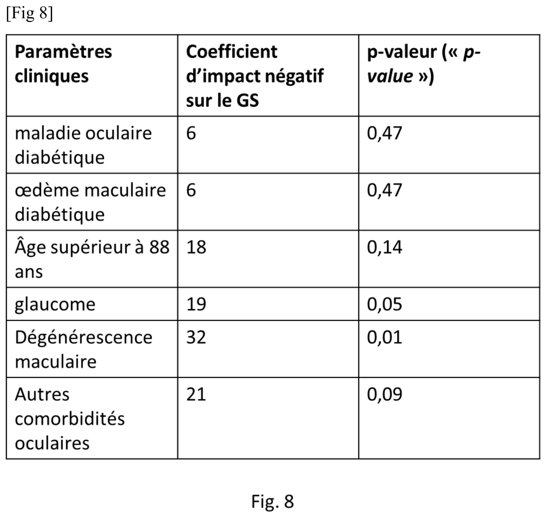

La figure 8 est un tableau regroupant les paramètres cliniques identifiés comme ayant un impact négatif sur le gain de santé, à partir de la population d'individus de l'exemple illustré dans les figures 6 et 7. Ainsi, la présence d'une dégénérescence maculaire a été identifiée comme ayant un impact négatif, statistiquement significatif, sur le gain de santé (p-valeur $\leq 0,05$). De même, la présence d'un glaucome a été identifiée comme ayant un impact négatif, statistiquement significatif, sur le gain de santé (p-valeur $\leq 0.05$).

Indice de risque

Un indice de risque peut être déterminé, à partir des paramètres cliniques identifiés comme ayant un impact négatif sur le gain de santé.

L'indice de risque peut être une somme pondérée, des coefficients d'impact négatif sur le gain de santé étant déterminés. Les coefficients d'impact négatif peuvent être choisis de sorte que plus un paramètre clinique a un impact négatif sur le gain de santé, statistiquement significatif, plus le coefficient d'impact négatif sur le gain de santé sera important.

L'indice de risque peut être calculé au moyen d'une régression linéaire multivariée. D'autres méthodes de calcul statistique peuvent être utilisées.

Cet indice de risque permet notamment de pouvoir identifier, pour un nouvel individu, la catégorie de profil de risque à laquelle il appartient. La population peut être enrichie.

Il est également possible d'identifier les types d'implants intraoculaires, et/ou les contextes opératoires, les plus adaptés en fonction de la catégorie de profil de risque. Ainsi, en déterminant à quelle catégorie un nouvel individu appartient, il est possible de proposer un implant intraoculaire, et/ou un contexte opératoire, adapté(s) au nouvel individu.

Calcul du gain de santé de référence

A l'étape C), pour chaque catégorie de profil de risque déterminée à l'étape de classification, un gain de santé de référence est calculé.

En particulier, ce gain de santé de référence (GS$_{réf}$) est égal à la médiane des gains de santé des individus de la catégorie de profil de risque.

Le gain de santé de référence peut alternativement être égal à la moyenne des gains de santé des individus de la catégorie de profil de risque.

Le gain de santé de référence peut être une combinaison, éventuellement une combinaison linéaire de la médiane des gains de santé des individus de la catégorie de profil de risque et de la moyenne des individus de la catégorie de profil de risque. Alternativement, un quantile différent de la moyenne et la médiane peut être utilisé pour déterminer le gain de santé. Une combinaison de quantiles peut être utilisée.

A l'issue de l'étape C) d'un procédé de comparaison tel qu'illustré à la figure 1, on compare les types d'implants intraoculaires et/ou les contextes opératoires en comparant les gains de santé, pour chaque catégorie de profil de risque considérée séparément.

En particulier, les types d'implants intraoculaires et/ou les contextes opératoires peuvent être ordonnées selon un ordre croissant, alternativement décroissant, de gains de santé, la liste ordonnée étant par exemple présentée au praticien.

La comparaison peut être affichée sur un écran 14.

La figure 2 illustre différentes étapes d'un procédé de comparaison d'implants et/ou de contextes opératoires selon l'invention comportant en outre les étapes D) à F).

Le procédé de comparaison selon l'invention est avantageusement mis en œuvre par ordinateur.

Les étapes A) à C) sont décrites ci-dessus.

Coefficients de pondération

A l'étape D), pour chaque catégorie de profil de risque, on détermine un coefficient de pondération.

Les coefficients de pondération sont choisis de telle sorte que :

[Math 2]

$$\forall i \in [\![1, N]\!], \forall k \in [\![1, N]\!], k \neq i,$$

$$c_i * GS_{réf,i} = c_k * GS_{réf,k} + \varepsilon \Leftrightarrow GS_{ajusté,i} = GS_{ajusté,k} + \varepsilon$$

GS$_{réf,i}$, étant le gain de santé de référence de la catégorie i, GS$_{ajusté,i}$ étant le gain de santé ajusté de la catégorie i, N étant le nombre de catégories déterminées à l'étape B), c$_i$ étant le coefficient de pondération de la catégorie i, $\varepsilon$ étant inférieur à 10% du maximum des gains de santé de référence, mieux inférieur à 5% du maximum des gains de santé de référence, mieux inférieur à 2% du maximum des gains de santé de référence, encore mieux $\varepsilon$ est nul.

Dans l'exemple des figures 6 et 7, on peut choisir les coefficients suivants c$_1$ = 1,22 ; c$_2$ = 1 ; c$_3$ = 5,54. On obtient alors :

[Math 3]

$$c_1 * GS_{réf,1} \approx c_2 * GS_{réf,2} \approx c_3 * GS_{réf,3} \Leftrightarrow 1,22 * 20,9 \approx 1 * 25,5 \approx 5,54 * 4,6$$

le gain de santé de référence (GS$_{réf}$) étant égal à la médiane de la catégorie.

D'autres coefficients peuvent être choisis.

Ajustement des gains de santé

A l'étape E), pour chaque individu de la population, on multiplie le gain de santé par le coefficient de pondération déterminé pour la catégorie à laquelle appartient l'individu, de sorte à obtenir un ensemble de gains de santé ajustés.

Par exemple, pour tous les individus de la première catégorie, le gain de santé sera multiplié par c$_1$, c'est-à-dire par 1,22, pour tous les individus de la deuxième catégorie, le gain de santé sera multiplié par c$_2$, c'est-à-dire 1, et pour tous les individus de la troisième catégorie, le gain de santé sera multiplié par $c_3$, c'est-à-dire 5,54.

Comparaison

A l'étape F), on compare les types d'implants intraoculaires et/ou les contextes opératoires en comparant les gains de santé ajustés.

En particulier, les types d'implants intraoculaires et/ou les contextes opératoires peuvent être ordonnées selon un ordre croissant, alternativement décroissant, de gains de santé ajustés, la liste ordonnée étant par exemple présentée au praticien.

La comparaison peut être affichée sur un écran 14.

Système de traitement de données

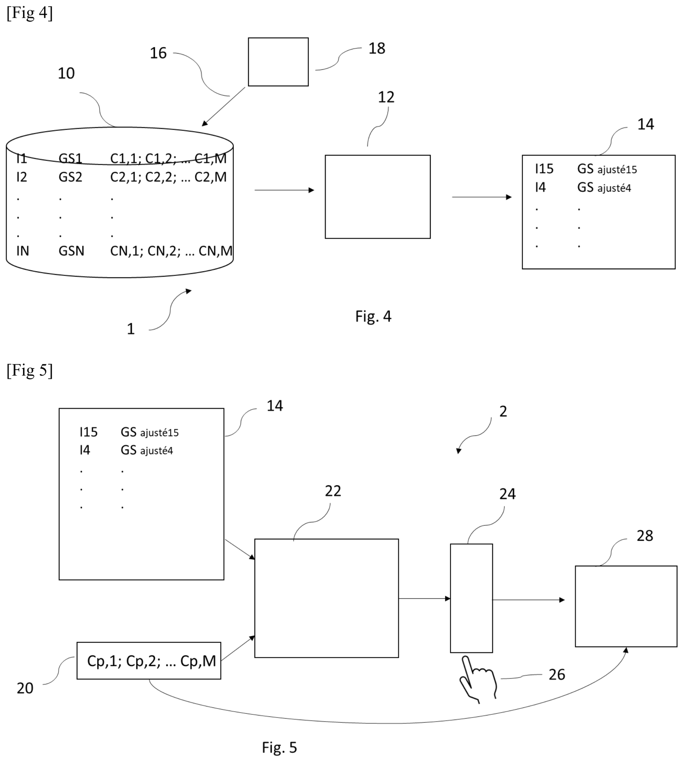

La figure 4 est un schéma bloc d'un système de traitement de données 1 pour la mise en œuvre d'un procédé de comparaison selon l'invention, comportant :

une base de données 10 stockant pour une population d'individus portant chacun un implant intraoculaire : au moins un type d'implants intraoculaires et/ou un paramètre du contexte opératoire, des paramètres cliniques et un gain de santé ;

un programme d'ordinateur 12 comprenant des instructions qui, lorsque le programme est exécuté par un ordinateur, conduisent celui-ci à mettre en œuvre les étapes B) à E) d'un procédé de comparaison selon l'invention.

Le système de traitement de données 1 peut comporter des moyens de communication 16 permettant de recueillir le type d'implants et/ou le paramètre du contexte opératoire, les paramètres cliniques et les gains de santé pour la mise en œuvre de l'étape A) d'un procédé de comparaison selon l'invention.

Les moyens de communication 16 peuvent être des moyens de communication digitale enrichissant la base de données à partir de renseignement fournis via une interface digitale 18.

Le système de traitement de données peut comporter un écran 14 pour présenter la comparaison de l'étape E).

Procédé de fabrication

La figure 3 représente les étapes d'un procédé de fabrication d'un implant intraoculaire.

Le procédé de fabrication comporte les étapes 1) et 2) d'un procédé d'aide à la sélection d'un type d'implants intraoculaires, suivi d'une étape de fabrication d'un implant choisi parmi les types d'implants sélectionnés.

Procédé de sélection

L'étape 1) consiste à fournir des résultats d'une comparaison d'implants, résultant de la mise en œuvre d'un procédé de comparaison tel que décrit précédemment pour comparer le bénéfice apporté par un type d'implants intraoculaires à un individu.

Dans l'exemple décrit, les résultats de la comparaison d'implants sont issus d'un procédé selon l'invention comportant les étapes A) à F).

L'étape 2) comporte le seuillage des résultats fournis à l'étape 1), de sorte à sélectionner les types d'implants intraoculaires pour lesquels les gains de santé ajustés sont supérieurs à une valeur seuil prédéterminée.

La valeur seuil prédéterminée peut être déterminée par un praticien. Elle peut être choisie en accord avec le patient. Une valeur minimale de seuil peut être imposée par l'hôpital ou la clinique du praticien.

Dans un mode de réalisation particulier, la valeur seuil est calculée en fonction de paramètres cliniques du patient pour lequel une intervention médicale est planifiée, en particulier de paramètres cliniques identifiés comme ayant un impact négatif sur le gain de santé.

Le procédé d'aide à la sélection peut comporter avant l'étape 2), le renseignement d'au moins un paramètre clinique du patient, l'étape 2) comportant les étapes suivantes :

2.1) présélection des types d'implants intraoculaires pour lesquels les paramètres cliniques des individus de la population d'individus correspondent au paramètre clinique renseigné pour le patient; puis,

2.2) seuillage des gains de santé ajustés, en sélectionnant parmi les types d'implants intraoculaires présélectionnés à l'étape 2.1), les types d'implants intraoculaires associés à un gain de santé ajusté supérieur à une valeur seuil prédéterminée.

La présélection à l'étape 2.1) peut être réalisée en calculant un indice de risque.

Fabrication d'implant

A l'étape 3), un type d'implants intraoculaires est fabriqué.

En particulier, lorsque plusieurs types d'implants sont sélectionnés à l'étape 2), le type d'implants ayant le gain de santé ajusté le plus élevé peut être fabriqué à l'étape 3).

Alternativement, lorsque plusieurs types d'implants sont sélectionnés, le type d'implants à fabriquer peut être choisi selon d'autres critères.

Dispositif

La figure 5 est un schéma bloc d'un dispositif 2 destiné à mettre en œuvre, entre autres, un procédé de sélection et de fabrication selon l'invention.

Le dispositif comporte une unité de traitement informatique 22, un module d'affichage 24 et des moyens de fabrication 28.

L'unité de traitement informatique 22 reçoit une comparaison 14 du bénéfice apporté par un type d'implants intraoculaires à un individu, la comparaison étant issue d'un procédé de comparaison selon l'invention. La comparaison peut en outre comporter les contextes opératoires.

L'unité de traitement informatique filtre ensuite par seuillage la comparaison 14 de sorte à ne sélectionner que les types d'implants ayant un gain de santé ajusté supérieur à une valeur seuil prédéterminée.

En particulier, des paramètres cliniques 20 d'un patient p sont renseignés et transmis à l'unité de traitement informatique.

La valeur seuil peut être déterminée au moyen des paramètres cliniques (20) du patient p.

Alternativement, les paramètres cliniques (20) du patient p, peuvent servir à effectuer une pré-sélection, l'unité de traitement informatique présélectionne dans un premier temps les types d'implants associés à des individus dont les paramètres cliniques sont similaires à ceux renseignés pour le patient p, puis dans un second temps, l'unité de traitement sélectionne les types d'implants ayant un gain de santé ajusté supérieur à la valeur seuil prédéterminée, les types d'implants étant sélectionnés parmi les types d'implants présélectionnés.

Les types d'implants sélectionnés sont présentés à un opérateur 26 au moyen d'un module d'affichage 24, de préférence au praticien, par exemple sur un écran.

Le dispositif 2 schématisé sur la figure 5 peut en outre mettre en œuvre un procédé de fabrication selon l'invention.

Lorsque plusieurs types d'implants sont sélectionnés, le type d'implants ayant le gain de santé ajusté le plus élevé est sélectionné par défaut pour être fabriqué par les moyens de fabrication 28. Alternativement, ou additionnellement, d'autres critères que le gain de santé peuvent permettre de sélectionner le type d'implants à fabriquer.

Lorsqu'aucun type d'implants intraoculaires n'est sélectionné, cela peut être indicatif d'une intervention médicale non nécessaire. Par exemple, si un gain de santé trop faible est sélectionné, l'apport de l'implant intraoculaire sera probablement négligeable, voir préjudiciable dans le cas d'un gain de santé négatif.

De préférence la fabrication de l'implant dépend des paramètres cliniques 20 du patient p.

Bien entendu, l'invention n'est pas limitée aux exemples qui viennent d'être décrits.

Comme précisé précédemment, les exemples sont détaillés pour le cas particulier d'un implant intraoculaire. Les exemples sont transposables pour un implant destiné à tout autre intervention médicale. Les exemples sont également transposables à tout type de prothèses et en particulier au cas d'une prothèse de la hanche.

Comme il apparait clairement à présent, l'invention permet de comparer des contextes opératoires et en particulier des types d'implants ou des types de prothèses, quel que soit l'individu portant l'implant, respectivement la prothèse, et quel que soit le praticien ayant pratiqué l'intervention médiale.

L'invention permet également de prendre en compte l'apport réel de l'intervention médicale et ainsi limiter les interventions non pertinentes.

L'expression « comportant un » doit être comprise comme « comprenant au moins un » en l'absence d'indication contraire.

**Revendications**

1. Procédé d'aide à la sélection d'un type d'implants ou de prothèses, et/ou d'un contexte opératoire, pour planifier une intervention médicale pour un patient, le procédé d'aide à la sélection comportant les étapes suivantes :

1) renseignement de résultats d'une comparaison de types d'implants ou de prothèses pour une catégorie d'individu donnée, et/ou de contextes opératoires de mise en place de ces implants, respectivement prothèses, les résultats étant issus d'un procédé de comparaison d'implants ou de prothèses, et/ou de contextes opératoires de mise en place de l'implant ou de la prothèse, à partir d'une population d'individus portant chacun un implant ou une prothèse, mis en œuvre par un ordinateur,
le procédé de comparaison comportant les étapes suivantes :

A) pour chaque individu de la population d'individus :

- renseignement du type d'implants ou de prothèses, et/ou d'au moins un paramètre du contexte opératoire de mise en place de l'implant ou de la prothèse;
- acquisition de paramètres cliniques; et
- détermination d'un score de gain de santé (GS) quantifiant un bénéfice apporté à l'individu par l'intervention médicale ;

B) classification des individus de la population d'individus de sorte à obtenir au moins deux catégories de profil de risque indicatives d'un rapport entre un bénéfice apporté par une intervention médicale et le risque encouru par la mise en œuvre d'une intervention médicale, la classification regroupant les individus en fonction de leur gain de santé et de leurs paramètres cliniques, notamment en fonction des paramètres cliniques ayant un impact négatif sur le gain de santé, la classification étant en particulier effectuée au moyen d'une régression statistique, notamment une régression linéaire multivariée ;
C) pour chaque catégorie de profil de risque, calcul d'un gain de santé de référence, le gain de santé de référence étant un quantile, notamment la moyenne ou la médiane, ou une mesure proportionnelle à la différence minimale d'importance clinique (« MCID ») des gains de santé des individus de la catégorie, ou toute combinaison de ceux-ci ;
le procédé comportant, après l'étape C), la comparaison, au sein de chaque catégorie de profils de risque, des types d'implants ou de prothèses et/ou des contextes opératoires en fonction des gains de santé,
D) pour chaque catégorie de profil de risque, détermination d'un coefficient de pondération puis calcul d'un gain de santé de référence ajusté égal au produit du coefficient de pondération par le gain de santé de référence,
les coefficients de pondération étant choisis de sorte à obtenir un ensemble de gains de santé de référence ajustés sensiblement égaux entre eux ;
E) pour chaque individu de la population d'individus, ajustement du gain de santé, déterminé à l'étape A), en multipliant le gain de santé par le coefficient de pondération déterminé pour la catégorie à laquelle appartient l'individu, de sorte à obtenir un ensemble de gain de santé pondérés ;
F) comparaison des types d'implants ou de prothèses, et éventuellement des contextes opératoires, en comparant les gains de santé ajustés.

2) seuillage des résultats issus de l'étape 1) de sorte à sélectionner le ou les types d'implants, respectivement prothèses, et/ou le ou les contextes opératoires pour lesquels les gains de santé sont supérieurs à une valeur seuil prédéterminée ;

les prothèses étant des audioprothèses, des prothèses orthopédiques, des prothèses mammaires, des prothèses dentaires ou des stents.

**2.** Procédé d'aide à la sélection selon la revendication précédente, dans lequel, à l'étape F) du procédé de comparaison, on compare les types d'implants ou de prothèses en fonction des gains de santé ajustés et en fonction de paramètres cliniques impactant négativement le gain de santé.

**3.** Procédé d'aide à la sélection selon la revendication précédente, les paramètres cliniques impactant négativement le gain de santé étant identifiés à l'étape B) du procédé de comparaison.

**4.** Procédé d'aide à la sélection selon l'une quelconque des revendications précédentes, dans lequel, à l'étape A) du procédé de comparaison, pour chaque individu de la population d'individus, le gain de santé est déterminé par comparaison d'un indicateur représentatif d'un état pré-interventionnel de l'individu avec un indicateur représentatif d'un état post-interventionnel de l'individu, de préférence le ou les indicateurs d'états pré-interventionnel et post-interventionnel étant déterminés au moyen d'au moins un questionnaire, en particulier renseigné par l'individu.

**5.** Procédé d'aide à la sélection selon l'une des revendications précédentes, dans lequel, à l'étape B) du procédé de comparaison, la classification est effectuée par mise en œuvre d'un algorithme de classification prenant en entrées les gains de santé et les paramètres cliniques de la population et fournissant en sortie au moins deux catégories de profil de risque, en particulier l'algorithme étant choisi parmi : algorithme de centres mobiles (ou « k-*means* »), arbre de décision, analyse en composante principale, méthode des plus proches voisins.

**6.** Procédé d'aide à la sélection selon l'une quelconque des revendications précédentes, les prothèses étant des prothèses de hanche et de genou.

**7.** Procédé de fabrication d'un implant ou d'une prothèse, comportant la mise en œuvre d'un procédé d'aide à la sélection selon l'une quelconque des revendications précédentes de sorte à sélectionner au moins un type d'implants ou de prothèse, puis la fabrication d'un implant ou d'une prothèse de même type que celui ou celle qui a été sélectionné(e), notamment identique.

**8.** Système de traitement de données pour la mise en œuvre d'un procédé d'aide à la sélection selon l'une quelconque des revendications 1 à 6, comportant :

- une base de données stockant pour une population d'individus portant chacun un implant ou une prothèse : au moins un type d'implants ou de prothèses, et éventuellement un paramètre du contexte opératoire, des paramètres cliniques et un gain de santé;
- un programme d'ordinateur comprenant des instructions qui, lorsque le programme est exécuté par un ordinateur, conduisent celui-ci à mettre en œuvre les étapes 1) et 2) d'un procédé d'aide à la sélection selon l'une quelconque des revendications 1 6, en particulier d'un type d'implants ou d'un type de prothèses.

[Fig 1]

| A) Acquisition données |
| B) Classification des individus en catégories de profil de risque |
| C) Calcul des GS de référence |

Fig. 1

[Fig 2]

| A) Acquisition données |
| B) Classification des individus en catégories de profil de risque |
| C) Calcul des GS de référence |
| D) Détermination de coefficients de pondération |
| E) Ajustement des GS pour obtenir les GS ajustés |
| F) Comparaison des types d'implants et/ou de contextes opératoires |

Fig. 2

[Fig 3]

| 1) Fourniture d'une comparaison de types d'implants intraoculaires |
| 2) Sélection de types d'implants par seuillage |
| 3) Fabrication d'un implant intraoculaire |

Fig. 3

[Fig 4]

Fig. 4

[Fig 5]

Fig. 5

[Fig 6]

|  | Cat1 | Cat2 | Cat3 |
|---|---|---|---|
| Total individus | 210 | 47 | 12 |
| Âge moyen | 70,4 | 72,0 | 72,6 |
| Âge supérieur à 88 ans | 2 | 0 | 2 |
| femmes | 121 | 27 | 7 |
| Opération des deux yeux | 115 | 26 | 1 |
| Facteurs techniques | 0 | 2 | 0 |
| Comorbidités oculaires | 0 | 26 | 10 |
| Opérations ophtalmiques antérieures | 0 | 15 | 2 |
| Variables de sévérité | 0 | 7 | 2 |
| GS moyen | 20,9 | 25,5 | 4,6 |

Fig. 6

[Fig 7]

Fig. 7

[Fig 8]

| Paramètres cliniques | Coefficient d'impact négatif sur le GS | p-valeur (« *p-value* ») |
|---|---|---|
| maladie oculaire diabétique | 6 | 0,47 |
| œdème maculaire diabétique | 6 | 0,47 |
| Âge supérieur à 88 ans | 18 | 0,14 |
| glaucome | 19 | 0,05 |
| Dégénérescence maculaire | 32 | 0,01 |
| Autres comorbidités oculaires | 21 | 0,09 |

Fig. 8